# EUROPEAN PATENT SPECIFICATION

(11) **EP 1 848 342 B1**
(45) Date of publication and mention of the grant of the patent: **26.12.2012**
(21) Application number: 06708161.2
(22) Date of filing: 09.02.2006
(51) Int. Cl.: A61B 10/00

(54) **BIOPSY DEVICE**
BIOPSIEVORRICHTUNG
DISPOSITIF DE BIOPSIE

(30) Priority: 11.02.2005 IT MO20050033
(43) Date of publication of application: 31.10.2007
(73) Proprietor: H.S. - Hospital Service - S.P.A., 04011 Aprilia (LT) (IT)
(72) Inventor: BELLUOMO, Armando, 00121 Roma (IT)
(74) Representative: Crugnola, Pietro
(86) International application number: PCT/EP2006/050817
(87) International publication number: WO 2006/084885

(56) References cited:
- US-A- 4 600 014
- US-A- 5 394 887
- US-A- 5 538 010
- US-B1- 6 592 530

## Description

The present invention relates to a biopsy device of the guillotine type, i.e. to an instrument for taking samples of fragments of organs or tissues from patients, to diagnose the nature of a pathological process. The biopsy device according to the present invention is suitable, in particular, for taking samples of soft and/or compressible tissues.

From the state of the art, biopsy devices of the so-called "guillotine" type are known, that comprise a substantially cylindrical mandrel, near the tip of which a housing is obtained - for example obtained by making a flattening along a portion of the mandrel - intended to receive the sample to be taken, and a hollow needle, or cannula, with a cutting tip, slidingly coupled outside said mandrel. The housing has dimensions such as to receive a sample of tissue of sufficient size for the histological examinations to be conducted thereupon. The mandrel and the hollow needle are fixed to a movable body that controls the respective displacements of mandrel and hollow needle. The movable body is inserted inside a grip that enables the operator to operate the biopsy device.

In order to conduct the biopsy, the device is introduced into the patient's body, with the mandrel retracted inside the hollow needle so that only the tip emerges. When the tip of the mandrel has reached the zone of the patient's body from which the sample has to be taken, the mandrel is made to emerge from the hollow needle by mutual axial sliding. In this way, a portion of the tissue surrounding the mandrel surrounds the housing obtained on the mandrel. At this point, the hollow needle is advanced until it covers said housing so that the cutting point of the hollow needle separates from the surrounding tissue, with a guillotine action, the portion of tissue that has penetrated into the aforementioned housing. Lastly, the device is extracted from the patient's body.

If the biopsy has been conducted on an infected tissue, during retraction of the device from the patient's body there is the risk that the so-called "seeding" phenomenon will occur. The word "seeding" literally means insemination and refers to the possibility of spreading infected cells inside other healthy tissues in the retraction of the needle that has conducted the biopsy.

In fact, when the hollow needle is advanced to separate a portion of tissue from the surrounding tissue and trap it in the mandrel housing, the tip of the mandrel, which has been in contact with possible infected cells, remains outside the hollow needle.

During extraction of the biopsy device from the patient's body, a certain number of cells belonging to the zone in which the biopsy was conducted and which have remained adhering to the tip of the mandrel and to the distal end of the hollow needle, in numbers of varying amount according to various factors (roughness, correct cannula-mandrel coupling, etc) can spread in non-infected tissues that are between the zone in which the biopsy was conducted and the skin of the patient.

Biopsy devices, still of the guillotine type, are known that have particular shapes and geometries that, however, have steps, recesses, tips or localised section variations that increase enormously the risk that the phenomenon of "seeding" will occur and therefore the risk of causing even lethal infections for the patient.

A further drawback that occurs in the performance of a biopsy consists of the fact that, if the mandrel, or the hollow needle, during their advance in the patient's body, meet excessive resistance, the entire device may move back which may not be perceived by the operator. This moving back causes incorrect positioning of the device in the patient's body, with the consequence that the taking of the tissue sample may occur in a zone other than the preset one, which makes it necessary to repeat the biopsy. Furthermore, during the phase of advancing of the hollow needle it may be the mandrel and the device used as grips that move back, which causes failed cutting of the tissue or an only partial cutting thereof.

As in general it is unlikely that the mandrel will encounter excessive resistance during penetration, because the mandrel is contained almost completely inside the hollow needle and only the end part thereof emerges from the hollow needle during advancing of the mandrel, this risk is particularly high for the hollow needle. In fact, the hollow needle has all or a good part of the external surface thereof immersed in the tissues; the external surface is therefore subjected to a more or less high pressure field, depending on the type of tissue. At the moment in which the hollow needle starts advancing, a static friction is created that depends on the pressure exerted on the needle by the surrounding tissue, by the static friction coefficient between needle and tissue and by the extent of the surface of the needle inside the tissue. In addition to this friction, which is usually called stiction, the hollow needle encounters during advancing a dynamic friction that differs from static friction by the different value of the dynamic friction coefficient with respect to the static friction coefficient. Both the static friction and the dynamic friction oppose the regular advance of the hollow needle and often produce the undesired effect of a moving back of the mandrel and of the body of the device. This effect is undoubtedly deleterious for the biopsy; in fact, as the hollow needle does not advance, there is no possibility of cutting the tissue and thus the biopsy is not successful and often has to be repeated.

US 6 592 530 B1 discloses a biopsy device comprising a body, a mandrel provided with housing intended to receive a sample of organic tissue, a hollow needle externally coaxial with the mandrel, said mandrel and said hollow needle being slidingly coupled with said body and being movable in a direction parallel to the respective longitudinal axes, a distal portion of said mandrel and of said hollow needle protrudes outside said body; the biopsy device further comprises a cannula externally coaxial with said hollow needle.

The cannula is a guide for the mandrel and the hollow needle, but is not designed to be inserted into the body of the patient when a biopsy is to be performed, but remains outside the patient's body during the taking of biopsy sample.

The mandrel and the hollow needle remain outside the cannula until they have been completely extracted from the body of the patient.

US 5 394 887 A discloses a stylet for a biopsy needle with a specimen recess inwardly of the distal end thereof which has a bottom wall parallel to the axis of the stylet and distal and proximal end walls which are reentrant relative to the distal end and proximal position of the stylet, respectively.

US 4 600 014 A, on which the preamble of claim 1 is based, discloses an improved biopsy needle suitable for obtaining a core sample from a prostate gland. A handle which may be held in the palm of the physician's hand is provided with a forward extending guide tube. A cannula with a sharpened distal end is slidably disposed within the guide tube and is movable by a thumb tab from a retracted position within the guide tube to an extended position in which the cutting edge extends from the guide tube. A sampling stylet having a sharpened distal end and a sample-collecting slot therein is telescopically disposed within the cannula and projects from the rear of the handle. A knob at the proximal end of the stylet permits movement from a retracted position in which the pointed tip of the stylet projects slightly from the distal end of the guide tube to an extended position in which the pointed distal end extends from the guide tube distal end. The physician holding the handle in the palm of his hand places his index finger at the distal end of the guide tube and guides the distal end to a selected point on the prostate gland transrectally. Upon locating the desired point, the hand holds the instrument steady while the other hand pushes the stylet forward into the tissue, pushes the cannula forward to sever tissue within the sampling slot, and the device is then withdrawn with the desired sample.

The present invention aims to remedy the drawbacks specified above.

According to the present invention a biopsy device for taking a sample of organic tissue is provided comprising a body, a mandrel provided with housing intended to receive a sample of organic tissue, a hollow needle externally coaxial with the mandrel and a cannula externally coaxial with said hollow needle, said mandrel and said hollow needle being designed to be inserted into said organic tissue, said mandrel and said hollow needle being slidingly coupled with said body and being movable in a direction parallel to the respective longitudinal axes between a respective proximal position and a respective distal position, a distal portion of said mandrel and of said hollow needle protruding outside said body, said cannula being designed to be inserted into said organic tissue together with said mandrel and said hollow needle, said cannula having a sufficient length to cover at least a substantial part of the distal portion of said hollow needle when the hollow needle is in said proximal position, said body comprising a first body part and a second body part between which there is defined a cavity, a proximal end of said mandrel being fixed to a first movable element suitable for sliding in said cavity in a direction substantially parallel to longitudinal axes of said mandrel and said hollow needle and further comprising driving means that is slidable inside said cavity in a direction substantially parallel to said longitudinal axes, characterised in that said driving means comprises a first groove orientated in a direction substantially parallel to said longitudinal axes and suitable for being coupled with a second appendage of said first movable element.

Owing to the presence of the cannula, when the hollow needle is advanced from the proximal position thereof to the distal position thereof, to separate a portion of tissue from the surrounding tissue and entrap it in the housing of the mandrel, only a short end portion of the hollow needle protrudes from the cannula and is subject to friction with the surrounding tissue, so that resistance to the advancing of the hollow needle is minimal and the risk of moving back of the device is substantially eliminated and, consequently, the risk of taking the bioptic sample in an incorrect position and also the risk of not taking a sufficient sample to conduct the subsequent analyses correctly are substantially eliminated.

In a further embodiment of the invention, the cannula is of sufficient length to cover completely both the distal portion of the hollow needle and the distal portion of the mandrel when they are both in the respective proximal positions.

This enables the mandrel and the hollow needle to be retracted completely inside the cannula, before extracting the device from the patient's body, so that possible infected cells that remain adhering to the mandrel and to the hollow needle remain inside the cannula whilst the device is extracted from the patient's body, thus making contamination of non-infected tissues impossible.

Another important advantage of the possibility of covering the distal ends of the hollow needle 8 and mandrel 6 is the elimination of the risk that the operator could hurt himself with the cutting surfaces of the mandrel or of the hollow needle that are presumed to be infected.

In a still further embodiment of the invention, the cannula is fixed in a removable manner to the body of the device. The possibility of separating the cannula from the body of the device makes it possible, after inserting the device into the patient's body and carrying out the biopsy, to leave the cannula in situ, so that it can act as a guide for carrying out further biopsies where they are necessary.

The invention will now be disclosed, purely by way of non-limitative example, with reference to the attached drawings, in which:
Figure 1 is a longitudinal section of an embodiment of the device according to the invention, in a first operating position;
Figure 2 is a longitudinal section of the device in Figure 1, in a second operating position;
Figure 3 is a longitudinal section of the device in Figure 1, in a third operating position;
Figure 4 is a longitudinal section of the device in Figure 1, in a fourth operating position;
Figures 5 to 8 are views from above of the device in Figures 1 to 4, respectively;
Figures 9 to 15 illustrate components of the device in Figures 1 to 4;
Figure 16 is a longitudinal section of a further embodiment of the device according to the invention, in a first operating position;
Figure 17 is a longitudinal section of the device in Figure 16, in a second operating position;
Figure 18 is a longitudinal section of the device in Figure 16, in a third operating position;
Figure 19 is a longitudinal section of the device in Figure 16, in a fourth operating position;
Figures 20 to 23 are views from above of the device in

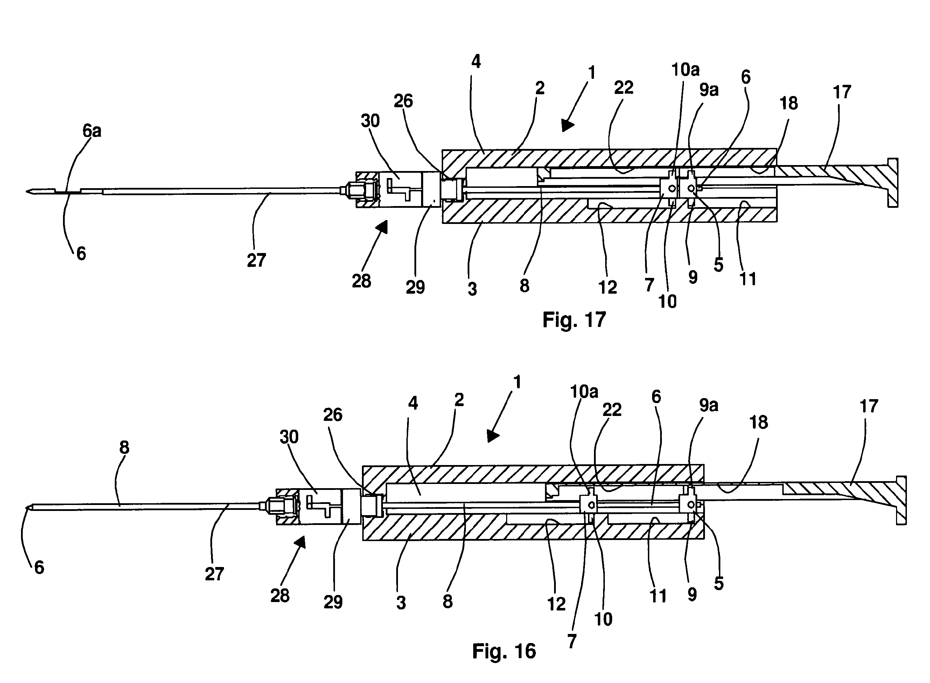

Figures 16 to 19, respectively;

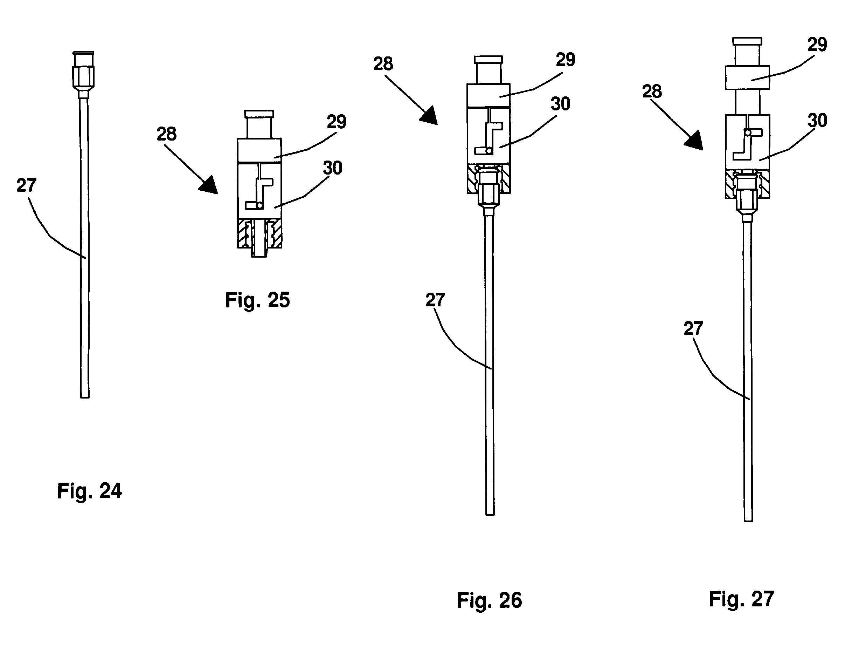

Figures 24 to 27 show details of the device in Figures 16 to 19.

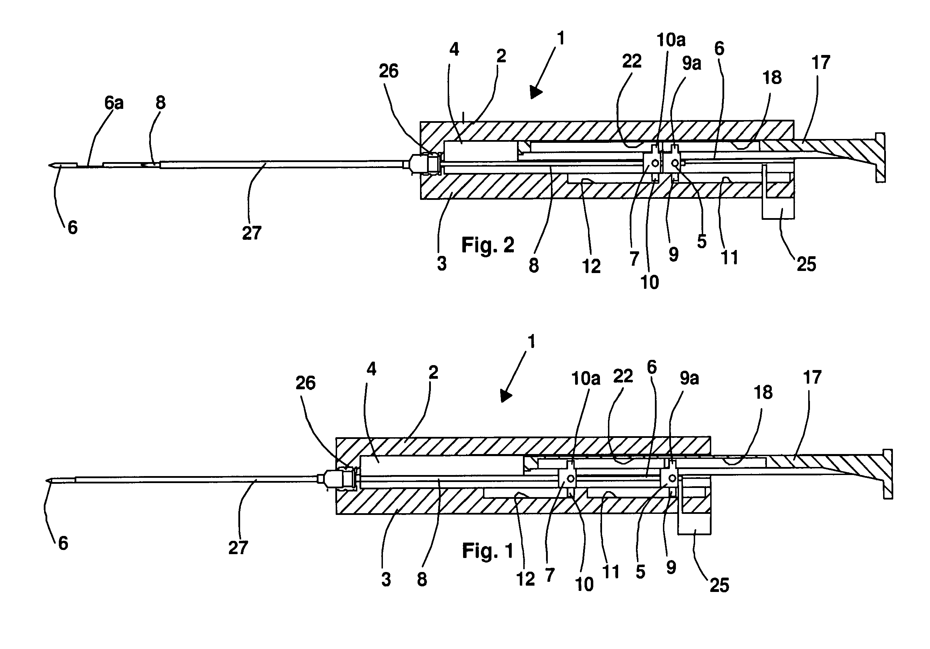

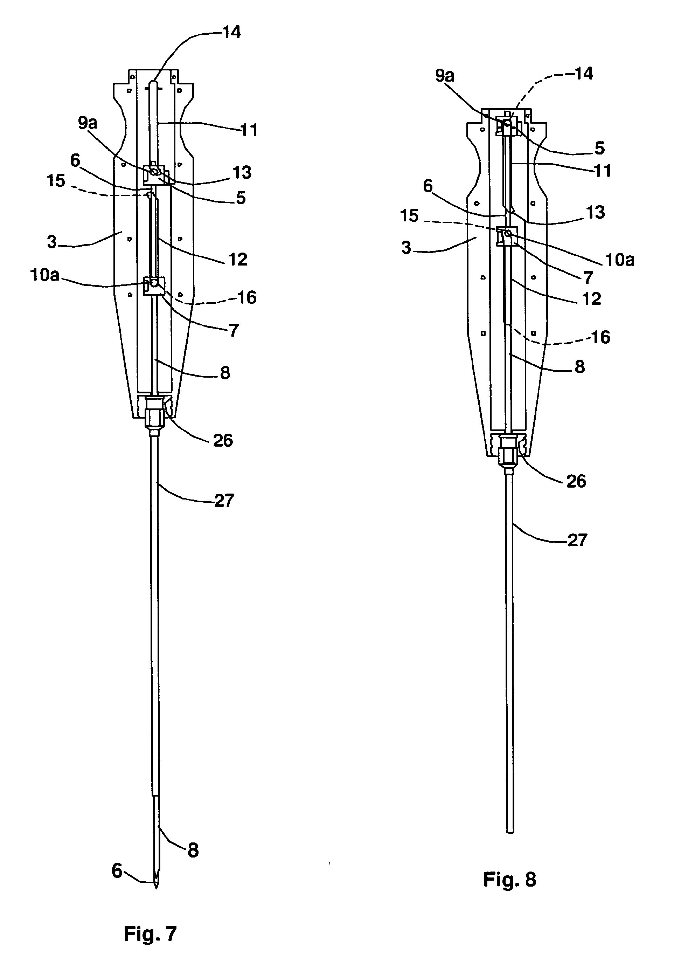

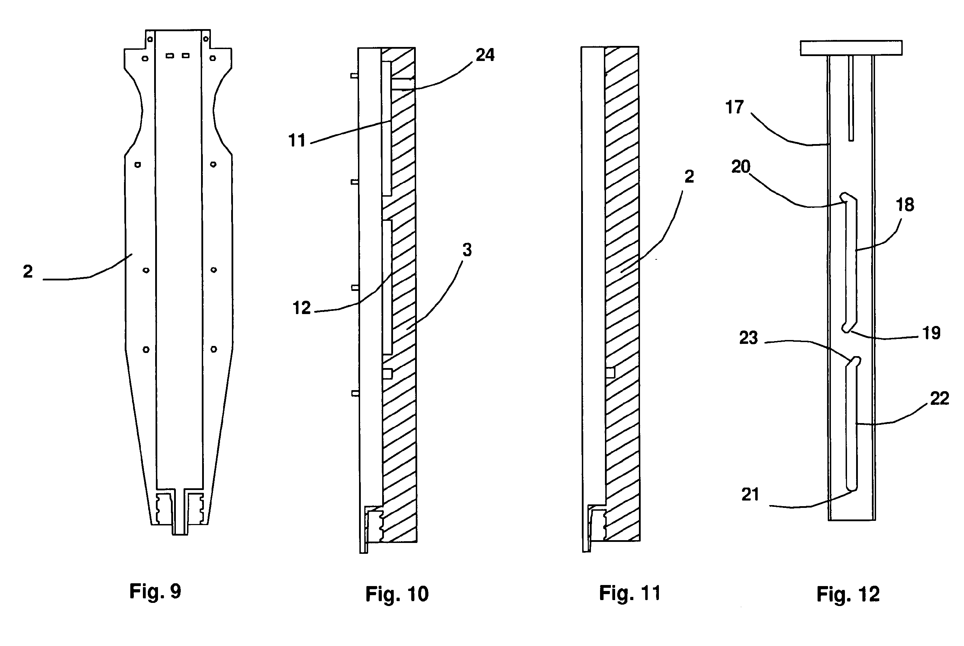

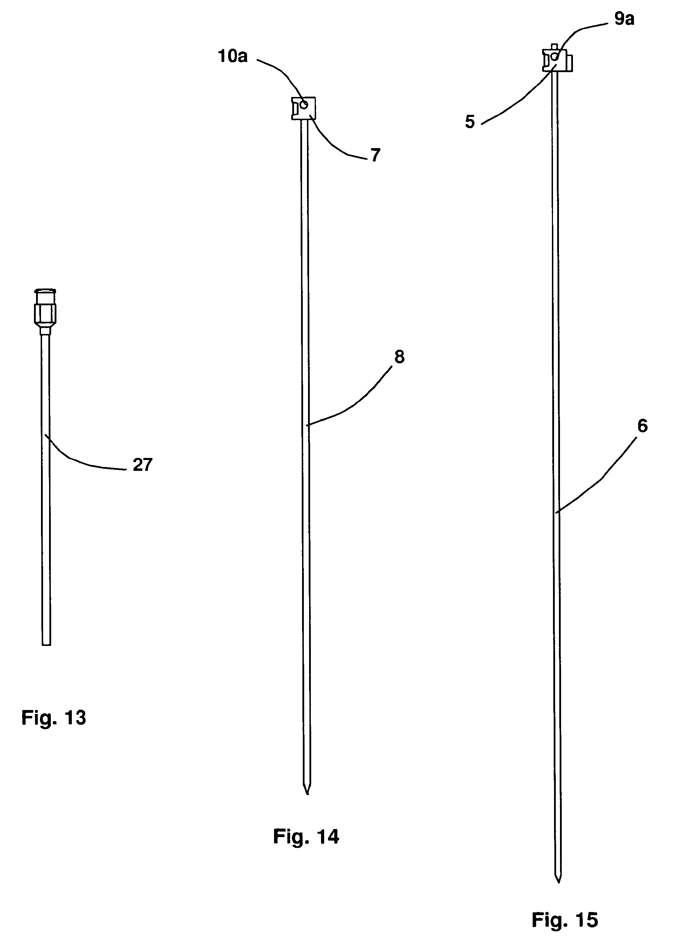

With reference to Figures 1 to 15, a biopsy device according to the invention comprises a body 1 consisting of a first body part 2 and a second body part 3, which are coupled together. Inside the body 1, between said first part 2 and said second part 3 a cavity 4 is defined inside which a first movable element 5 is slidable to which there is fixed a proximal end of a mandrel 6, and a second movable element 7 to which a proximal end of a hollow needle 8 is fixed that surrounds the mandrel 6 externally.

The mandrel 6 is provided, near the proximal end thereof, with a seat 6a intended to receive a sample of organic tissue.

The first movable element 5 and the second movable element 7 are slidable in a direction substantially parallel to a longitudinal axis of the mandrel 6 and of the hollow needle 8.

The first movable element 5 is provided with a first appendage 9 and with a second appendage 9a, opposite said first appendage 9. The appendages 9, 9a extend in a direction substantially perpendicular to said longitudinal axis.

The second movable element 7 is also provided with a first appendage 10 and with a second appendage 10a, opposite said first appendage 10. The appendages 10, 10a extend in a direction substantially perpendicular to said longitudinal axis.

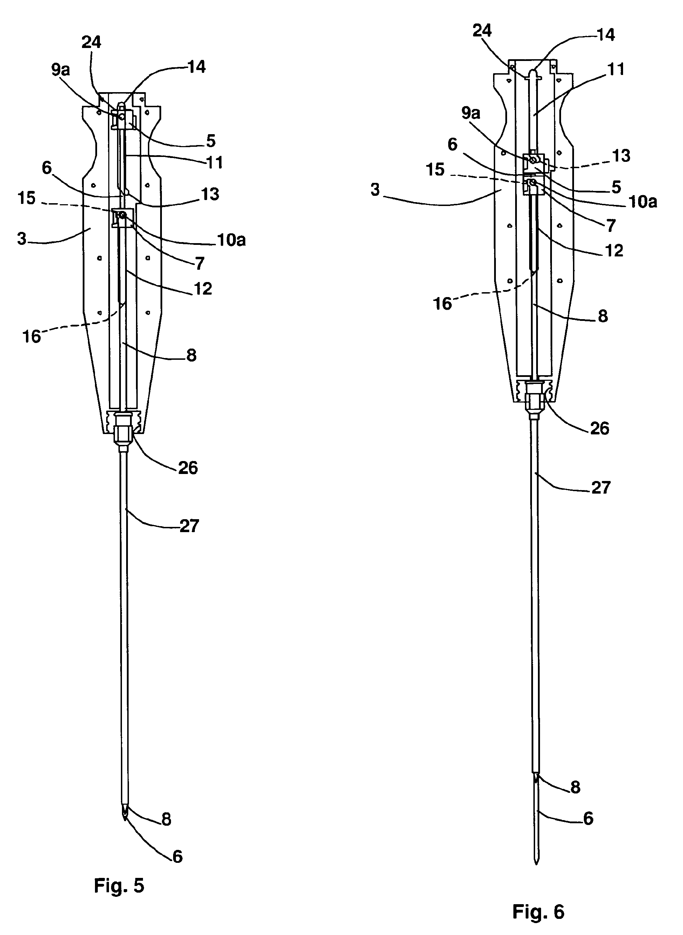

In the second body part 3 a first groove 11 and a second groove 12 are obtained that are both communicating with said cavity 4 and aligned in a direction substantially parallel to said longitudinal axis. The first groove 11 is intended to be coupled with the first appendage 9 of the first movable element 5 during the movements of the latter inside the cavity 4. The first groove 11 terminates, at a distal end thereof, with a notch 13 (fig. 5), offset laterally with respect to said longitudinal axis, intended to receive and lock said first appendage 9, in a distal position of the first movable element 5, that defines a distal position of the mandrel 6. An end 14 of the groove 11 opposite said notch 13 defines a proximal stop position for the first movable element 5 that defines a proximal position of the mandrel 6. The second groove 12 is intended to couple with the first appendage 10 of the second movable element 7 during the movement of the latter inside the cavity 4. The second groove 12 terminates, at the proximal end thereof, with a notch 15, offset laterally with respect to said longitudinal axis, intended to receive and lock said first appendage 10, in a proximal stop position of the second movable element 7, that defines a proximal position of the hollow needle 8. An end 16 of the groove 12, opposite said portion 15, defines a distal stop position for the second movable element 7 that defines a distal position of the hollow needle 8.

The first movable element 5 and the second movable element 7 are drivable to slide inside the cavity 4 by a driving element 17 that is slidingly couplable, as a piston, inside the cavity 4.

The driving element 17 is provided with a first groove 18 that extends in a direction substantially parallel to said longitudinal axis and is intended to be coupled with the second appendage 9a of the first movable element 5. The first groove 18 terminates, at a distal end thereof, with a first notch 19, offset laterally with respect to said longitudinal axis, intended to guide said first movable element 5 to the distal stop position previously mentioned. The first groove 18 furthermore terminates at a proximal end thereof with a second notch 20, offset laterally with respect to said longitudinal axis, intended to guide said first movable element 5 to the proximal stop position mentioned previously.

The driving element 17 is furthermore provided with a second groove 22 substantially aligned with said first groove 18 in said direction parallel to said longitudinal axis. The second groove 22 is intended to be coupled with the second appendage 10a of the second movable element 7 and terminates at a proximal end thereof with a notch 23 offset laterally with respect to said longitudinal axis, intended to guide said second movable element 7 to the distal stop position previously mentioned.

In the second part 3 of the body 1 a through hole 24 is made, orientated in a direction substantially perpendicular to said longitudinal axis. In the opening 24 a stop element 25 is slidable that defines an intermediate stop position for the first movable element 5, between said proximal stop position and said distal stop position. The distance of the intermediate stop position from the proximal stop position of the movable element 5 is preferably less than the distance of the intermediate stop position from the distal stop position of the movable element 5.

At said distal end of the body 1 a seat 26 is obtained in which it can be fixed in a removable manner, for example with a coupling of the so-called "luer-lock" type, a proximal end of a cannula 27 that externally surrounds the mandrel 6 and the hollow needle 8, being coaxial with them (the coupling between the seat 26 and a proximal end of a cannula 27 can also be envisaged fixed and obtained through a simple luer cone glued to the surfaces of the seat). The length of the cannula 27 is chosen so that it is able to cover completely a distal portion of hollow needle 8 that protrudes from the body 1, when the hollow needle 8 is in the proximal position thereof.

In order to conduct a biopsy, the device 1 is initially in a first operating position, illustrated in Figure 1, in which the first movable element 5 is in the intermediate stop position against the stop element 25 and the second movable element 7 is in the proximal stop position, with the first appendage 10 engaged and locked in the notch 15 of the second groove 12 of the second element 3 of the body 1. The driving element 17 is positioned so that the second appendage 9a of the first movable element 5 is engaged and locked in the first notch 19 of the first groove 18.

In this first operating position, the distal portion of hollow needle 8 that protrudes from the body 1 is completely inside the cannula 27 from which only the tip of the mandrel 6 protrudes.

With the device in this first operating position, the mandrel 6, the hollow needle 8 and the cannula 27 are introduced into the body of a patient until the distal end of the mandrel 6 reaches the zone in which the biopsy has to be conducted.

When the distal end of the mandrel 6 has reached the zone in which the biopsy has to be conducted, the driving element 17 is advanced to the distal end of the body 1, which causes an advance to said distal end of the first movable element 5, inasmuch as the second appendage 9a thereof, locked in the first notch 19 of the first groove 18 of the driving element 17, is dragged by the latter, thus causing the advance of the first movable element 5. Advancing of the driving element continues until the first appendage 9 of the first movable element 5 reaches the distal end of the first groove 11 of the lower part 3 of the body 2, engaging and locking in the notch 13.

Advancing the first movable element 5 causes advancing of the mandrel 6 that emerges from the cannula 27 until it completely uncovers the seat 6a, so as to enable a portion of organic tissue to surround said seat 6a (Fig. 2).

At this point, the driving element 17 is advanced further to the distal end of the body 1 of the device. This further advancing causes the disengagement of the second appendage 9a of the first movable element 5 from the first notch 19 of the first groove 18, whilst the second appendage 10a of the second movable element 7 engages and remains locked in the notch 23 of the second groove 22 of the driving element 17. In this way, the further advance of the driving element 17 determines a corresponding advance of the second movable element 7, whilst the first movable element 5 remains stationary in the distal stop position.

Advancing of the driving element 17 and of the second movable element 7 terminates when the first appendage 10 of the second movable element 7 reaches the distal end of the second groove 12 of the second part 3 of the body 1.

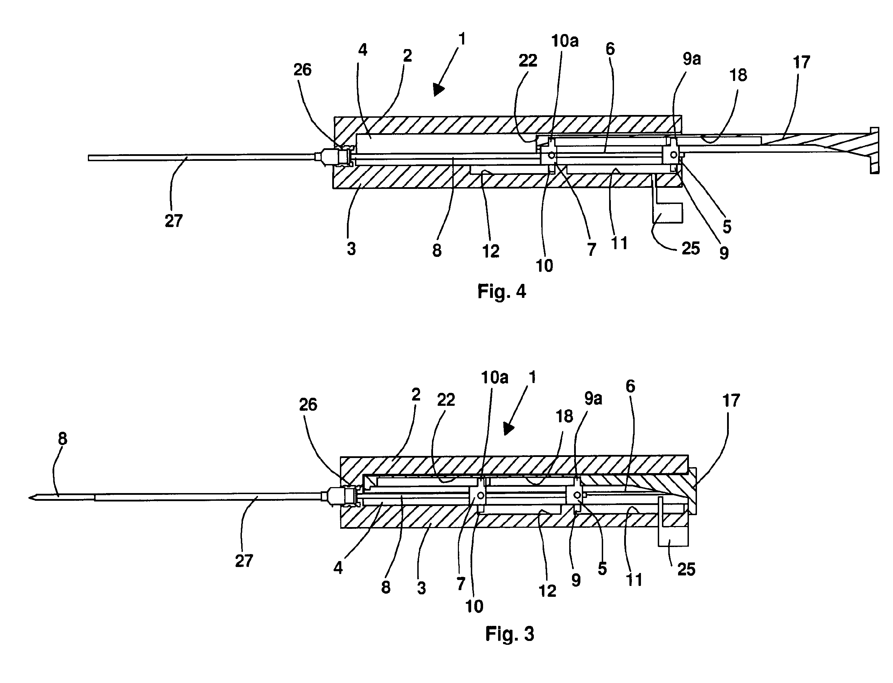

The advancing of the second movable element 7 causes corresponding advancing of the hollow needle 8, the distal end of which emerges from the cannula 27 and advances until it cover completely the seat 6a of the mandrel 6 (Fig. 3). A portion of tissue that surrounds the seat 6a is cut by the distal end of the hollow needle 8 and remains trapped in the seat 6a, inside the hollow needle 8.

Before extracting the device according to the invention from the patient's body, the stop element 25 moves towards the outside of the body 2, so that it no longer interferes with the first movable element 5. The driving element 17 is then moved away from the distal end of the body 2 until the second movable element 7 is brought to its proximal stop position and the first movable element 5 is brought to its proximal stop position, as shown in Figure 4. This causes simultaneous movement of the mandrel 6 and of the hollow needle 8 towards the proximal end of the body 2 until they reach the respective proximal positions, in which the distal ends of the mandrel 6 and of the hollow needle 8 are both completely inside the cannula 27 (Fig. 4).

In this condition, the device can be extracted from the patient's body without the risk that possible infected cells coming from the zone of the biopsy and remaining adhering to the distal end of the mandrel 6 and/of the hollow needle 8 may contaminate healthy tissues that are located along the extraction trajectory of the device from the patient's body. This is because the cannula 27 completely shields the distal ends of the mandrel 6 and of the hollow needle 8, preventing said possible infected cells being able to be deposited on healthy tissues.

The cannula 27 furthermore protects the operator from the risk of coming into contact with cutting surfaces presumed to be infected.

During this phase, in the event of removable coupling, it is possible to decouple the cannula 27 from the device, leaving it in situ in the patient's body to act as a guide if further bioptic samples need to be taken from the same zone of the patient's body.

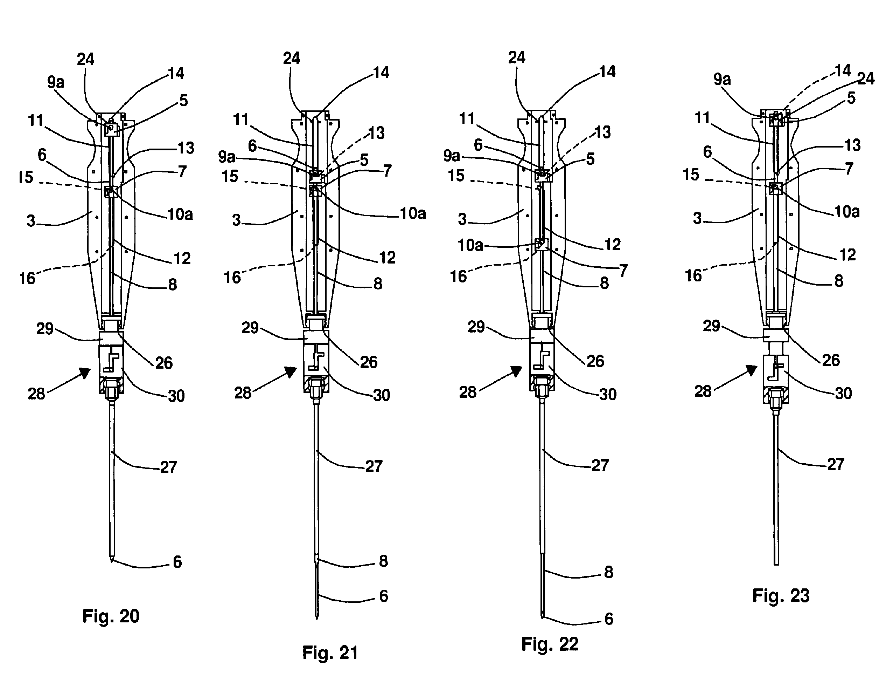

In Figures 16 to 27 there is illustrated another embodiment of a device according to the invention, in which covering of the distal ends of the mandrel 6 and of the hollow needle 8 is achieved by making the cannula 27 movable in a direction parallel to the longitudinal axis of the mandrel and of the hollow needle.

In this embodiment of the device the stop element 25 is not present, so no intermediate stop position of the movable element 5 is defined between the distal stop position thereof and the proximal stop position thereof. The cannula 27 is coupled in a removable manner, through, for example, a coupling of the "luer-lock" type to an operating element 28 fixed to the seat 26 of the body 2, the latter also, for example, with coupling of the "luer-lock" type (also in this case the above couplings can be made fixed, for example through gluing). The operating element 28 consists of a first part 29, fixed to said seat 26 and of a second part 30 slidingly coupled with said first part, so as to be able to slide in relation to it in a direction substantially parallel to said longitudinal axis, so as to vary the length of the operating element 28 and consequently, the position of the cannula 27 in relation to the mandrel 6 and to the hollow needle 8.

The operation of this embodiment is illustrated in Figures 16 to 23.

In order to carry out a biopsy, the device 16 starts in a first operating position, illustrated in Figure 1, in which both the first movable element and the second movable element 7 are in the respective proximal stop positions with the first appendage 10 of the second movable element 7 engaged and locked in the notch 15 of the second groove 12 of the second element 3 of the body 1. The driving element 17 is positioned so that the second appendage 9a of the first movable element 5 is engaged and locked in the first notch 19 of the first groove 18.

In this first operating position, the distal portion of hollow needle 8 that protrudes from the body 1 is completely inside the cannula 27, from which only the tip of the mandrel 6 protrudes. In this first operating position it is also possible to provide for the end part of the distal portion of the hollow needle, at the cutting surfaces of the latter element, protruding outside the surfaces of the cannula 27.

With the device in this first operating position, the mandrel 6, the hollow needle 8 and the cannula 27 are inserted into the body of a patient until the distal end of the mandrel 6 reaches the zone in which the biopsy has to be conducted.

When the distal end of the mandrel 6 has reached the zone in which the biopsy has to be conducted, the driving element 17 is advanced towards the distal end of the body 1, which causes an advance towards said distal end of the first movable element 5, inasmuch as the second appendage 9a thereof, locked in the first notch 19 of the first groove 18 of the driving element 17, is dragged by the latter, thus causing the advance of the first movable element 5. The advance of the driving element continues until the first appendage 9 of the first movable element 5 reaches the distal end of the first groove 11 of the lower part 3 of the body 2, engaging and locking in the notch 13.

Advancing of the first movable element 5 causes an advance of the mandrel 6 that emerges from the cannula 27 until it uncovers the seat 6a completely, so as to enable a portion of organic tissue to penetrate into said seat 6a (Fig. 17). At this point, the driving element 17 is advanced further towards the distal end of the body 1 of the device. This further advance causes the disengagement of the second appendage 9a of the first movable element 5 from the first notch 19 of the first groove 18, whilst the second appendage 10a of the second movable element 7 engages and remains locked in the notch 23 of the second groove 22 of the driving element 17. In this way, the further advance of the driving element 17 causes a corresponding advance of the second movable element 7, whilst the first movable element 5 remains stationary in the distal stop position.

The advancing of the driving element 17 and of the second movable element 7 terminates when the first appendage 10 of the second movable element 7 reaches the distal end of the second groove 12 of the second part 3 of the body 1.

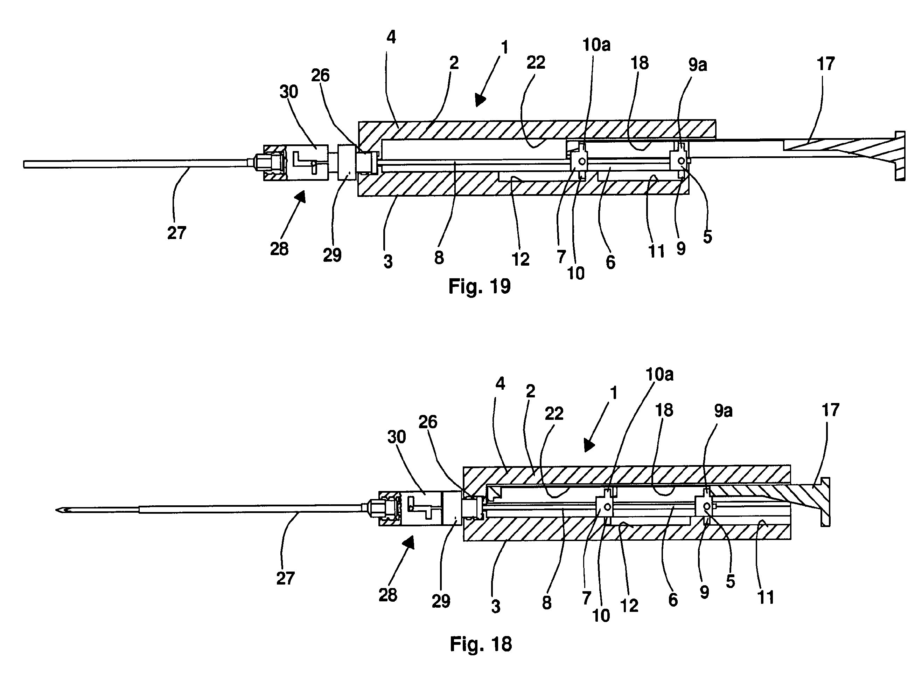

The advance of the second movable element 7 causes a corresponding advance of the hollow needle 8, the distal end of which emerges from the cannula 27 and advances until it cover completely s the seat 6a of the mandrel 6 (Fig. 18). A portion of tissue that surrounds the seat 6a is cut by the distal end of the hollow needle 8 and remains entrapped in the seat 6a, inside the hollow needle 8.

Before extracting the device according to the invention from the patient's body, it is necessary to release the operating device 28 so that the two elements constituting it, i.e. the element 30 integral with the cannula 27 and the element 29 integral with the body 1, have the possibility of moving in relation to one another. After the release, it is necessary to make the body 1 move back, keeping the element 30 stationary until the element 29 reach the distal stop obtained on the element 30. With this operation it is possible to insert the mandrel 6 and the hollow needle 8 (Figure 19) completely inside the cannula 27.

In this condition the device can be extracted from the patient's body without the risk that possible infected cells coming from the zone of the biopsy and remaining adhering to the near end of the mandrel 6 and/or of the hollow needle 8 may contaminate the healthy tissues that are located along the extraction trajectory of the device from the patient's body.

In the practical embodiment, the materials, dimensions and constructional details may be different from those indicated but without thereby falling outside the scope of the present invention, as defined by the claims.

## Claims

1. Biopsy device for taking a sample of organic tissue, comprising a body (1), a mandrel (6) provided with housing (6a) intended to receive a sample of organic tissue, a hollow needle (8) externally coaxial with the mandrel and a cannula (27) externally coaxial with said hollow needle (8), said mandrel (6) and said hollow needle (8) being designed to be inserted into said organic tissue, said mandrel (6) and said hollow needle (8) being slidingly coupled with said body (1) and being movable in a direction parallel to the respective longitudinal axes between a respective proximal position and a respective distal position, a distal portion of said mandrel (6) and of said hollow needle (8) protruding outside said body (1), said cannula (27) being designed to be inserted into said organic tissue together with said mandrel (6) and said hollow needle (8) during the taking of said sample, said cannula (27) having a sufficient length to cover at least a substantial part of the distal portion of said hollow needle (8) when the hollow needle (8) is in said proximal position, said body (1) comprising a first body part (2) and a second body part (3) between which there is defined a cavity (4), a proximal end of said mandrel (6) being fixed to a first movable element (5) suitable for sliding in said cavity (4) in a direction substantially parallel to said longitudinal axes of said mandrel (6) and said hollow needle (8), the biopsy device further comprising driving means (17) that is slidable inside said cavity (4) in a direction substantially parallel to said longitudinal axes, **characterised in that** said driving means (17) comprises a first groove (18) orientated in a direction substantially parallel to said longitudinal axes and being coupled with a second appendage (9a) of said first movable element (5) for sliding longitudinal movement of the driving means relative to the first movable element.

2. Device according to claim 1, wherein said cannula (27) has a sufficient length to cover completely the distal portion of said hollow needle (8) and of said mandrel (6), when both are in the respective proximal positions.

3. Device according to claim 1, or 2, wherein said cannula (27) is removably coupled with said body (1).

4. Device according to claim 1, or 2, wherein said cannula (27) is irremovably coupled with said body (1).

5. Device according to any preceding claim, wherein said first movable element (5) is provided with a first appendage (9) and with said second appendage (9a) both extending in a direction substantially perpendicular to said longitudinal axes.

6. Device according to any preceding claim, wherein a proximal end of said hollow needle (8) is fixed to a second movable element (7) suitable for sliding in said cavity (4) in a direction substantially parallel to said longitudinal axes.

7. Device according to claim 6, wherein said second movable element (7) is provided with a first appendage (10) and with a second appendage (10a) both extending in a direction substantially perpendicular to said longitudinal axes.

8. Device according to claim 5 , wherein said second part (3) of said body (2) is provided with a first groove (11) suitable for being coupled with the first appendage (9) of the first movable element (5).

9. Device according to claims 8 and 6, wherein said second part (3) of said body (2) is provided with a second groove (12) suitable for being coupled with the first appendage (10) of the second movable element (7), said second groove (12) being aligned on said first groove (11) in a direction substantially parallel to said longitudinal axes.

10. Device according to claim 9, wherein said first groove (11) of said second body part is provided, at a distal end thereof, with a notch (13) offset laterally with respect to said direction.

11. Device according to claim 9, or 10, wherein said second groove (12) of said second body part is provided, at a proximal end thereof, with a notch (15) offset laterally with respect to said direction.

12. Device according to any preceding claim, wherein said driving means (17) comprises a second groove (22) aligned with said first groove (18) of said driving means and suitable for being coupled with the second appendage (10a) of said second movable element (7).

13. Device according to any preceding claim, wherein said first groove of said driving means is provided, at a distal end thereof with a first notch (19) offset laterally with respect to said direction.

14. Device according to any preceding claim, wherein said first groove (18) of said driving means is provided at a proximal end thereof with a second notch (20) offset laterally with respect to said direction.

15. Device according to any preceding claim, wherein said second groove (22) of said driving means is provided, at a proximal end thereof, with a notch (23) offset laterally with respect to said direction.

16. Device according to any preceding claim, wherein with said second part (3) of said body (2) there is associated a stop element (25) suitable for interacting with said first movable element (5).

17. Device according to claim 16, wherein said stop element (25) is slidable in an opening (24) of said second body part (3), in a direction substantially perpendicular to said longitudinal axes.

18. Device according to claim 1, or any one of claims 3 to 17, wherein said cannula is coupled with said body (1) by means of an operating element (28) of variable length.

19. Device according to claim 18, wherein said operating element (28) comprises a first part (29) coupled with a seat (26) provided in said body (1) and a second part (30) slidingly coupled with said first part (29), so as to be able to slide with respect to it in a direction substantially parallel to said longitudinal axes between a proximal position and a distal position, said cannula (27) being coupled with said second part (30).

20. Device according to claim 19, wherein said cannula (27) has sufficient length to cover completely said hollow needle (8) and said mandrel (6) when both are in the respective proximal positions and said second part (30) of the operating element (28) is in the respective distal position.

## Patentansprüche

1. Biopsievorrichtung zum Entnehmen einer Probe von organischem Gewebe, mit einem Körper (1), einem Mandrin (6), der mit einem Gehäuse (6a) versehen ist, das eine Probe von organischem Gewebe aufnehmen soll, einer Hohlnadel (8), die außen zum Mandrin koaxial ist, und einer Kanüle (27), die außen zur Hohlnadel (8) koaxial ist, wobei der Mandrin (6) und die Hohlnadel (8) so ausgelegt sind, dass sie in das organische Gewebe eingeführt werden, wobei der Mandrin (6) und die Hohlnadel (8) mit dem Körper (1) verschiebbar gekoppelt sind und in einer zu den jeweiligen Längsachsen parallelen Richtung zwischen einer jeweiligen proximalen Position und einer jeweiligen distalen Position beweglich sind, wobei ein distaler Abschnitt des Mandrins (6) und der Hohlnadel (8) aus dem Körper (1) vorsteht, wobei die Kanüle (27) so ausgelegt ist, dass sie in das organische Gewebe zusammen mit dem Mandrin (6) und der Hohlnadel (8) während der Entnahme der Probe eingeführt wird, wobei die Kanüle (27) eine ausreichende Länge aufweist, um zumindest einen wesentlichen Teil des distalen Abschnitts der Hohlnadel (8) zu bedecken, wenn sich die Hohlnadel (8) in der proximalen Position befindet, wobei der Körper (1) einen ersten Körperteil (2) und einen zweiten Körperteil (3) umfasst, zwischen denen ein Hohlraum (4) definiert ist, wobei ein proximales Ende des Mandrins (6) an einem ersten beweglichen Element (5) befestigt ist, das zum Gleiten in dem Hohlraum (4) in einer Richtung, die zu den Längsachsen des Mandrins (6) und der Hohlnadel (8) im Wesentlichen parallel ist, geeignet ist, wobei die Biopsievorrichtung ferner eine Antriebseinrichtung (17) umfasst, die innerhalb des Hohlraums (4) in einer Richtung verschiebbar ist, die zu den Längsachsen im Wesentlichen parallel ist, **dadurch gekennzeichnet, dass** die Antriebseinrichtung (17) eine erste Nut (18) umfasst, die in einer zu den Längsachsen im Wesentlichen parallelen Richtung orientiert ist und mit einem zweiten Ansatz (9a) des ersten beweglichen Elements (5) für eine Gleitlängsbewegung der Antriebseinrichtung relativ zum ersten beweglichen Element gekoppelt ist.

2. Vorrichtung nach Anspruch 1, wobei die Kanüle (27) eine ausreichende Länge aufweist, um vollständig den distalen Abschnitt der Hohlnadel (8) und des Mandrins (6) zu bedecken, wenn sich beide in den jeweiligen proximalen Positionen befinden.

3. Vorrichtung nach Anspruch 1 oder 2, wobei die Kanüle (27) mit dem Körper (1) entfernbar gekoppelt ist.

4. Vorrichtung nach Anspruch 1 oder 2, wobei die Kanüle (27) mit dem Körper (1) nicht entfernbar gekoppelt ist.

5. Vorrichtung nach einem vorangehenden Anspruch, wobei das erste bewegliche Element (5) mit einem ersten Ansatz (9) und mit dem zweiten Ansatz (9a) versehen ist, die sich beide in einer zu den Längsachsen im Wesentlichen senkrechten Richtung erstrecken.

6. Vorrichtung nach einem vorangehenden Anspruch, wobei ein proximales Ende der Hohlnadel (8) an einem zweiten beweglichen Element (7) befestigt ist, das zum Gleiten in dem Hohlraum (4) in einer zu den Längsachsen im Wesentlichen parallelen Richtung geeignet ist.

7. Vorrichtung nach Anspruch 6, wobei das zweite bewegliche Element (7) mit einem ersten Ansatz (10) und mit einem zweiten Ansatz (10a) versehen ist, die sich beide in einer zu den Längsachsen im Wesentlichen senkrechten Richtung erstrecken.

8. Vorrichtung nach Anspruch 5, wobei der zweite Teil (3) des Körpers (2) mit einer ersten Nut (11) versehen ist, die zum Koppeln mit dem ersten Ansatz (9) des ersten beweglichen Elements (5) geeignet ist.

9. Vorrichtung nach den Ansprüchen 8 und 6, wobei der zweite Teil (3) des Körpers (2) mit einer zweiten Nut (12) versehen ist, die zum Koppeln mit dem ersten Ansatz (10) des zweiten beweglichen Elements (7) geeignet ist, wobei die zweite Nut (12) auf die erste Nut (11) in einer zu den Längsachsen im Wesentlichen parallelen Richtung ausgerichtet ist.

10. Vorrichtung nach Anspruch 9, wobei die erste Nut (11) des zweiten Körperteils an einem distalen Ende davon mit einer Kerbe (13) versehen ist, die in Bezug auf die Richtung seitlich versetzt ist.

11. Vorrichtung nach Anspruch 9 oder 10, wobei die zweite Nut (12) des zweiten Körperteils an einem proximalen Ende davon mit einer Kerbe (15) versehen ist, die in Bezug auf die Richtung seitlich versetzt ist.

12. Vorrichtung nach einem vorangehenden Anspruch, wobei die Antriebseinrichtung (17) eine zweite Nut (22) umfasst, die auf die erste Nut (18) der Antriebseinrichtung ausgerichtet ist und zum Koppeln mit dem zweiten Ansatz (10a) des zweiten beweglichen Elements (7) geeignet ist.

13. Vorrichtung nach einem vorangehenden Anspruch, wobei die erste Nut der Antriebseinrichtung an einem distalen Ende davon mit einer ersten Kerbe (19) versehen ist, die in Bezug auf die Richtung seitlich versetzt ist.

14. Vorrichtung nach einem vorangehenden Anspruch, wobei die erste Nut (18) der Antriebseinrichtung an einem proximalen Ende davon mit einer zweiten Kerbe (20) versehen ist, die in Bezug auf die Richtung seitlich versetzt ist.

15. Vorrichtung nach einem vorangehenden Anspruch, wobei die zweite Nut (22) der Antriebseinrichtung an einem proximalen Ende davon mit einer Kerbe (23) versehen ist, die in Bezug auf die Richtung seitlich versetzt ist.

16. Vorrichtung nach einem vorangehenden Anspruch, wobei dem zweiten Teil (3) des Körpers (2) ein Anschlagelement (25) zugeordnet ist, das zum Zusammenwirken mit dem ersten beweglichen Element (5) geeignet ist.

17. Vorrichtung nach Anspruch 16, wobei das Anschlagelement (25) in einer Öffnung (24) des zweiten Körperteils (3) in einer zu den Längsachsen im Wesentlichen senkrechten Richtung verschiebbar ist.

18. Vorrichtung nach Anspruch 1 oder irgendeinem der Ansprüche 3 bis 17, wobei die Kanüle mit dem Körper (1) mittels eines Bedienelements (28) mit variabler Länge gekoppelt ist.

19. Vorrichtung nach Anspruch 18, wobei das Bedienelement (28) einen ersten Teil (29), der mit einem Sitz (26) gekoppelt ist, der im Körper (1) vorgesehen ist, und einen zweiten Teil (30), der verschiebbar mit dem ersten Teil (29) gekoppelt ist, so dass er in Bezug auf diesen in einer zu den Längsachsen im Wesentlichen parallelen Richtung zwischen einer proximalen Position und einer distalen Position gleiten kann, umfasst, wobei die Kanüle (27) mit dem zweiten Teil (30) gekoppelt ist.

20. Vorrichtung nach Anspruch 19, wobei die Kanüle (27) eine ausreichende Länge aufweist, um die Hohlnadel (8) und den Mandrin (6) vollständig zu bedecken, wenn sich beide in den jeweiligen proximalen Positionen befinden und der zweite Teil (30) des Bedienelements (28) sich in der jeweiligen distalen Position befindet.

## Revendications

1. Dispositif de biopsie pour prélever un échantillon de tissu organique, comprenant un corps (1), un mandrin (6) doté d'un logement (6a) destiné à recevoir un échantillon de tissu organique, une aiguille creuse (8) extérieurement coaxiale avec le mandrin et une canule (27) extérieurement coaxiale avec ladite aiguille creuse (8), ledit mandrin (6) et ladite aiguille creuse (8) étant conçus pour être insérés dans ledit tissu organique, ledit mandrin (6) et ladite aiguille creuse (8) étant couplés par coulissement audit corps (1) et étant mobiles dans une direction parallèle aux axes longitudinaux respectifs entre une position proximale respective et une position distale respective, une partie distale dudit mandrin (6) et de ladite aiguille creuse (8) dépassant à l'extérieur dudit corps (1), ladite canule (27) étant conçue pour être insérée dans ledit tissu organique conjointement audit mandrin (6) et à ladite aiguille creuse (8) pendant le prélèvement dudit échantillon, ladite canule (27) ayant une longueur suffisante pour couvrir au moins une partie substantielle de la partie distale de ladite aiguille creuse (8) lorsque l'aiguille creuse (8) se trouve dans ladite position proximale, ledit corps (1) comprenant une première partie de corps (2) et une seconde partie de corps (3) entre lesquelles est définie une cavité (4), une extrémité proximale dudit mandrin (6) étant fixée à un premier élément mobile (5) adapté à coulisser dans ladite cavité (4) dans une direction sensiblement parallèle auxdits axes longitudinaux dudit mandrin (6) et de ladite aiguille creuse (8), le dispositif de biopsie comprenant en outre des moyens d'entraînement (17) qui peuvent coulisser à l'intérieur de ladite cavité (4) dans une direction sensiblement parallèle auxdits axes longitudinaux, **caractérisé en ce que** lesdits moyens d'entraînement (17) comprennent une première rainure (18) orientée une direction sensiblement parallèle auxdits longitudinaux et étant couplée à un second appendice (9a) dudit premier élément mobile (5) pour un mouvement longitudinal coulissant des moyens d'entraînement relativement au premier élément mobile.

2. Dispositif selon la revendication 1, dans lequel ladite canule (27) a une longueur suffisante pour couvrir complètement la partie distale de ladite aiguille creuse (8) et dudit mandrin (6), lorsque les deux se trouvent dans les positions proximales respectives.

3. Dispositif selon la revendication 1 ou 2, dans lequel ladite canule (27) est couplée de manière amovible audit corps (1) .

4. Dispositif selon la revendication 1 ou 2, dans lequel ladite canule (27) est couplée de manière non amovible audit corps (1).

5. Dispositif selon l'une quelconque des revendications précédentes, dans lequel ledit premier élément mobile (5) est doté d'un premier appendice (9) et dudit second appendice (9a), s'étendant tous les deux dans une direction sensiblement perpendiculaire auxdits axes longitudinaux.

6. Dispositif selon l'une quelconque des revendications précédentes, dans lequel une extrémité proximale de ladite aiguille creuse (8) est fixée à un second élément mobile (7) adapté à coulisser dans ladite cavité (4) dans une direction sensiblement parallèle auxdits axes longitudinaux.

7. Dispositif selon la revendication 6, dans lequel ledit second élément mobile (7) est doté d'un premier appendice (10) et d'un second appendice (10a), s'étendant les deux dans une direction sensiblement perpendiculaire auxdits axes longitudinaux.

8. Dispositif selon la revendication 5, dans lequel ladite seconde partie (3) dudit corps (2) est dotée d'une première rainure (11) adaptée à être couplée au premier appendice (9) du premier élément mobile (5).

9. Dispositif selon les revendications 8 et 6, dans lequel ladite seconde partie (3) dudit corps (2) est dotée d'une seconde rainure (12) adaptée à être couplée au premier appendice (10) du second élément mobile (7), ladite seconde rainure (12) étant alignée sur ladite première rainure (11) dans une direction sensiblement parallèle auxdits axes longitudinaux.

10. Dispositif selon la revendication 9, dans lequel ladite première rainure (11) de ladite seconde partie de corps est dotée, à une extrémité distale de celle-ci, d'une encoche (13) décalée latéralement relativement à ladite direction.

11. Dispositif selon la revendication 9 ou 10, dans lequel ladite seconde rainure (12) de ladite seconde partie de corps est dotée, à une extrémité proximale de celle-ci, d'une encoche (15) décalée latéralement relativement à ladite direction.

12. Dispositif selon l'une quelconque des revendications précédentes, dans lequel lesdits moyens d'entraînement (17) comprennent une seconde rainure (22) alignée avec ladite première rainure (18) desdits moyens d'entraînement et adaptée à être couplée au second appendice (10a) dudit second élément mobile (7).

13. Dispositif selon l'une quelconque des revendications précédentes, dans lequel ladite première rainure desdits moyens d'entraînement est dotée, à une extrémité distale de celle-ci, d'une première encoche (19) décalée latéralement relativement à ladite direction.

14. Dispositif selon l'une quelconque des revendications précédentes, dans lequel ladite première rainure (18) desdits moyens d'entraînement est dotée, à une extrémité proximale de celle-ci, d'une seconde encoche (20) décalée latéralement relativement à ladite direction.

15. Dispositif selon l'une quelconque des revendications précédentes, dans lequel ladite seconde rainure (22) desdits moyens d'entraînement est dotée, à une extrémité proximale de celle-ci, d'une encoche (23) décalée latéralement relativement à ladite direction.

16. Dispositif selon l'une quelconque des revendications précédentes, dans lequel est associé à ladite seconde partie (3) dudit corps (2) un élément d'arrêt (25) adapté à interagir avec ledit premier élément mobile (5) .

17. Dispositif selon la revendication 16, dans lequel ledit élément d'arrêt (25) est capable de coulisser dans une ouverture (24) de ladite seconde partie de corps (3), dans une direction sensiblement perpendiculaire auxdits axes longitudinaux.

18. Dispositif selon la revendication 1, ou l'une quelconque des revendications 3 à 17, dans lequel ladite canule est couplée audit corps (1) au moyen d'un élément fonctionnel (28) de langueur variable.

19. Dispositif selon la revendication 18, dans lequel ledit élément fonctionnel (28) comprend une première partie (29) couplée à un siège (26) fourni dans ledit corps (1) et une partie (30) couplée par coulissement à ladite première partie (29), de manière à pouvoir coulisser relativement à celui-ci dans une direction sensiblement parallèle auxdits axes longitudinaux entre une position proximal et une position distale, ladite canule (27) étant couplée à ladite seconde partie (30) .

20. Dispositif selon la revendication 19, dans lequel ladite canule (27) a une longueur suffisante pour couvrir complètement ladite aiguille creuse (8) et ledit mandrin (6) lorsqu'ils sont tous les deux dans les positions proximales respectives et ladite seconde partie (30) de l'élément fonctionnel (28) se trouve d0ans la position distale respective.
